# EUROPEAN PATENT SPECIFICATION

(11) **EP 4 086 635 B1**
(45) Date of publication and mention of the grant of the patent: **28.08.2024**
(21) Application number: 21172015.6
(22) Date of filing: 04.05.2021
(51) Int. Cl.: G01N 35/04, G01N 1/02

(54) **A PREANALYTIC SYSTEM FOR PREPARING A LABORATORY SAMPLE CONTAINER**
PRÄANALYTISCHES SYSTEM ZUR HERSTELLUNG EINES LABORPROBENBEHÄLTERS
SYSTÈME DE PRÉANALYSE POUR PRÉPARER UN RÉCIPIENT D'ÉCHANTILLONS DE LABORATOIRE

(43) Date of publication of application: 09.11.2022
(73) Proprietor: Roche Diagnostics GmbH, 68305 Mannheim (DE); F. Hoffmann-La Roche AG, 4070 Basel (CH)
(72) Inventor: Chotzidis, Angelos, 70806 Kornwestheim (DE); Soenthes, Stefan, 70806 Kornwestheim (DE); van Mierlo, Hans, 70806 Kornwestheim (DE)
(74) Representative: Altmann Stößel Dick Patentanwälte PartG mbB

(56) References cited:
- EP-A1- 2 282 212
- WO-A1-2018/005239
- US-A- 5 493 849
- US-A1- 2019 344 257

## Description

### Technical Field

The present invention relates to a diagnostic analyzer.

### Background art

In vitro diagnostic testing has a major effect on clinical decisions, providing physicians with pivotal information. Particularly, there is great emphasis on providing quick and accurate test results in critical care settings. In vitro diagnostic testing is usually performed by apparatus such as diagnostic analyzers using instruments operable to execute one or more processing steps or workflow steps on one or more biological samples and/or one or more reagents, such as pre-analytical instruments, post-analytical instruments and also analytical instruments.

Diagnostic instruments or analyzers are configured to obtain a measurement value from a sample. A diagnostic analyzer is operable to determine via various chemical, biological, physical, optical or other technical procedures a parameter value of the sample or a component thereof. A diagnostic analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. A diagnostic analyzer may comprise units assisting with the pipetting, dosing, and mixing of samples and/or reagents.

The diagnostic analyzer may comprise a process and detection system whose workflow is optimized for certain types of analysis. Examples of such analyzers are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

US 2019/344257 A1 discloses a preanalytic system according to the preamble of claim 1.

In laboratories, some types of instruments and/or analysis require closed laboratory sample containers such as sample tubes to be prepared such as opened before laboratory samples are pretreated and/or analyzed. For example, it may be required to handle so called swab collection tubes, for analysing bacteria or viruses. Such swab collection tubes have a swab that is connected to the cap of the tube. Since in most assays, the virus needs to be inactivated for the safety of the laboratory personnel and an inactivation or lysis fluid needs to be added to the sample to open up the cells so the virus comes out and can be measured. With handling such swab collection tubes, usually the removal of the cap and the addition of the inactivation or lysis fluid has to be done manually. With airborne viruses this procedure has to be done under the biosafety level 2 conditions with protective measures, i.e. laminar flow safety cabinets, protective clothes, gloves and masks. This makes it a very manual tedious process.

It is therefore desirable to provide a preanalytic system configured to at least minimize or even overcome the aforementioned drawbacks. Particularly, it is an aim of the present disclosure to provide a preanalytic system configured to decrease the number of manual process steps required for handling such laboratory sample containers such as opening and inactivating the sample contained therein.

### Summary

This problem is addressed by a preanalytic system with the features of the independent claim. Advantageous embodiments are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way.

According to a first aspect of the present disclosure, a preanalytic system for preparing a laboratory sample container is disclosed. The preanalytic system comprises a cap gripper configured to grip a cap of a laboratory sample container, wherein the cap is connected to a rod of a swab located within the laboratory sample container. The cap gripper is further configured to release the cap from the laboratory sample container. The preanalytic system further comprises a cutting device configured to cut the rod of the swab when the cap is in position released from the laboratory sample container.

With the above-described construction of the automatic analyzer, the cap gripper is configured to release the cap from the laboratory sample container. Thus, the cap is withdrawn from the laboratory sample container and spatially separated therefrom. In this released position, the cutting device cuts the rod while the cap is still gripped by the cap gripper. After cutting, the swab can slide into the sample in the laboratory sample container. The cap can be removed like any normal cap and wasted through the waste funnel into a biohazard disposal bag. Thus, the handling of the laboratory sample container including the opening and preparation for further processing is carried out in an automated manner without the need of any manual handling. thereby, also the biohazard risk for the laboratory personnel is significantly reduced.

The cap gripper may be further configured to release the cap from the laboratory sample container with the swab remaining located within the laboratory sample container. Thus, the cap of the tube needs only be released until a very short distance of approximately 2-3mm above the laboratory sample container which reduces the space necessary for the devices for removing the cap. Further, the swab can slide back into the sample in the laboratory sample container which reduces the risk of any spillage and contamination.

The cutting device may be configured to cut the rod of the swab such that the swab together with the cut rod remains located within the laboratory sample container. This reduces the risk of any spillage and contamination.

The laboratory sample container may extend along a longitudinal direction, wherein the cap gripper may be moveable in a direction parallel to the longitudinal direction. Thus, the cap may be released by a rather simple linear retraction movement of the cap gripper.

The cap gripper may be moveable in the direction parallel to the longitudinal direction with the cap gripped so as to lift the cap from the laboratory sample container.

The cap gripper may comprise at least two gripping fingers configured to grip the cap. Needless to say, the cap gripper may comprise three, four or even more gripping fingers. As such fingers comprise a rather longitudinal shape, the cap can be reliably and tightly gripped.

The gripping fingers may be configured to grip the cap at an outer circumferential surface thereof. Thus, the cap can be tightly gripped.

The cutting device may be rotatable, pivotable, linearly moveable or stationary relative to the cap gripper. Thus, the cutting process may be carried out by rather simple movements of the cutting device which reduces the space necessary for installation of the cutting device.

The cutting device may be a laser, a cutting wire and/or a cutting knife, particularly a heated cutting knife. Thus, the cutting device can be a device that can cut the swab without cleaning needs. Such a heated cutting knife may be heated to 60 degree Celsius or above, preferably between 60 and 70 degree or between 65 and 85 degree which temperature allows to degenerate biological material adhering on the blade of the cutting knife.

The preanalytic system may further comprise a container holder configured to hold the laboratory sample container. Thus, the laboratory sample container may be may be fixed in its position for decapping.

The container holder may be disposed at a position opposite to the cap gripper if seen along a common axial direction. Thus, the decapping or releasing of the cap may be carried out by a linear movement of the cap gripper relative to the container holder.

The preanalytic system may further comprise a waste container, wherein the cap gripper may be configured to dispose the cap cut off the swab into the waste container. Thus, the cap can be removed like any normal cap and wasted into a waste container which preferably includes a biohazard disposal bag.

The waste container may comprise a funnel having a wide end and a narrow end, wherein the funnel may be arranged such that the wide end faces the cap gripper. The cap will be removed like any normal cap and wasted through the waste funnel into a waste container which preferably includes biohazard disposal bag.

The waste container may be located below the cap gripper if seen in a direction of gravity. Thus, no further conveying means for transporting the cap is required. Rather, the cap may be released by the cap gripper and fall into the waste container.

The preanalytic system may further comprise an aliquoting device configured to take an aliquot from a sample of the laboratory sample container. Thus, an exact portion of the sample may be taken from the laboratory sample container.

The aliquoting device may be configured to dispense the aliquot from the sample of the laboratory sample container into an aliquot container. Thus, the aliquot may be used for further processing such as any analyzing processes.

The preanalytic system may further comprise a dispensing device configured to dispense an inactivation fluid and/or lysis fluid into the laboratory sample container. Since in most assays a virus needs to be inactivated for the safety of the laboratory personnel, an inactivation and/or lysis fluid needs to be added to the sample to open op the cells so the virus comes out and can be measured.

The dispensing device may be configured to dispense a predetermined amount of the inactivation fluid and/or lysis fluid into the laboratory sample container. This allows to pump a pre-defined volume of the inactivation fluid and/or lysis fluid into the sample container such as the aliquot container after aliquoting. It has to be noted that this is not a quantitative process as the fluid will not influence the result. However, the amount need to be a sufficient volume that will ensure a certain volume within the median margins for inactivation of the aliquotated sample such as a virus.

The dispensing device may comprise a pump, particularly a roller pump, configured to pump the predetermined amount of the inactivation fluid and/or lysis fluid into the laboratory sample container. Such a pump is a well-established device for exact dosage of any fluids.

The preanalytic system may further comprise a sealing device or recapping device configured to provide the laboratory sample container with a sealing or a cap. Thus, the laboratory sample container can be sealed for sorting into a waste rack on an output area of the preanalytic system.

The preanalytic system may further comprise a sterilizing device configured to sterilize the laboratory sample container. Thus, safety of the handled laboratory sample container may be increased for the laboratory personnel.

The sterilizing device may comprise an UV light source. Thus, sterilizing may be reliably carried out. Needless to say, also predetermined areas within the preanalytic system may be sterilized by means of such an UV light source increasing the safety for the laboratory personnel.

The term "preanalytic system" as used herein is a broad term and is to be given its ordinary and customary meaning to a person of ordinary skill in the art and is not to be limited to a special or customized meaning. The term specifically may refer, without limitation, to any apparatus, device or combination of apparatus and devices configured to prepare a laboratory sample container for further processing by a diagnostic analyzer. The preparation of the laboratory sample container may particularly be carried out in a diagnostic laboratory.

The term "preparing" and its equivalents as used herein is a broad term and is to be given its ordinary and customary meaning to a person of ordinary skill in the art and is not to be limited to a special or customized meaning. The term specifically may refer, without limitation, to the ways in which a sample is treated prior to its analyses. With other words, preparing refers to at least one processing step carried out at natural and/or synthetic samples for subsequent analysis thereof. Preparation is a very important step in most analytical techniques, because the techniques are often not responsive to the analyte in its in-situ form, or the results are distorted by interfering species. Sample preparation may involve dissolution, extraction, reaction with some chemical species, pulverizing, treatment with a chelating agent (e.g. EDTA), masking, filtering, dilution, sub-sampling, opening of a laboratory sample container or many other techniques. Treatment is done to prepare the sample into a form ready for analysis by specified analytical equipment such as a diagnostic analyzer. Sample preparation could involve: crushing and dissolution, chemical digestion with acid or alkali, sample extraction, sample clean up and sample pre-concentration.

The term "diagnostic analyzer" as used herein is a broad term and is to be given its ordinary and customary meaning to a person of ordinary skill in the art and is not to be limited to a special or customized meaning. The term specifically may refer, without limitation, to any apparatus or apparatus component operable to execute one or more processing steps/workflow steps on one or more biological samples. The term "processing step" thereby refers to physically executed processing steps such as centrifugation, aliquotation, sample analysis and the like. The term "analyzer" covers pre-analytical sample work-cells, post-analytical sample work-cells and also analytical work-cells. Non-limiting examples for diagnostic analyzers are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term "gripping device" as used herein is a broad term and is to be given its ordinary and customary meaning to a person of ordinary skill in the art and is not to be limited to a special or customized meaning. The term specifically may refer, without limitation, to any device that is configured to hold something such as an object very tightly. Particularly, the object may be a laboratory sample container. For this purpose the gripping device may comprise two or more gripping fingers. A gripping finger refers to any opposable articulated structure for grasping something. For this purpose, gripping fingers can open and close relative to one another.

The term "laboratory sample container" as used herein is a broad term and is to be given its ordinary and customary meaning to a person of ordinary skill in the art and is not to be limited to a special or customized meaning. The term specifically may refer, without limitation, to a device designed as a tube. The laboratory sample container is typically made of glass or transparent plastic and typically has an opening at an upper end. The container can be used to contain, store and transport a laboratory sample such as a blood sample, an urine sample or any other chemical sample.

The term "cap" as used herein is a broad term and is to be given its ordinary and customary meaning to a person of ordinary skill in the art and is not to be limited to a special or customized meaning. The term specifically may refer, without limitation, to a device that is typically used to close or seal the laboratory sample container, in particular its opening, and is typically embodied separate from the container. Depending on the laboratory sample and the container, the cap may have several functions. One may be to keep the container closed and the sample contained for the specified shelf life until time of opening. Another one may be to provide a barrier, e.g. to dirt, oxygen and/or moisture. The cap may comprise rubber and/or plastic or may completely consist of rubber and/or plastic. Further, the cap has typically a cylindrical shape, in particular with a circular cross section. Furthermore, the laboratory sample container and/or the cap have/has typically means of attaching to each other with sufficient security, e.g. threads, locks and/or adhesives may be used.

The term "cutting device" as used herein is a broad term and is to be given its ordinary and customary meaning to a person of ordinary skill in the art and is not to be limited to a special or customized meaning. The term specifically may refer, without limitation, to any device configured to separate the cap of a laboratory sample container from a rod of a swab. The term more specifically may refer, without limitation, to any device configured to separate the cap of a laboratory sample container from a rod of a swab by means of a cutting process.

The term "swab" as used herein is a broad term and is to be given its ordinary and customary meaning to a person of ordinary skill in the art and is not to be limited to a special or customized meaning. The term specifically may refer, without limitation, to an item that comprises a small wad of cotton wrapped around one end of a short rod made of wood, rolled paper, or plastic. Medical-type swabs are often used to take microbiological cultures. Pharmaceutical grade cotton swabs are necessary for medical applications. These medical grade swabs are rubbed onto or into the infected area, then wiped across the culture medium, such as an agar plate, where bacteria from the swab may grow. They are also used to take DNA samples, most commonly by scraping cells from the inner cheek in the case of humans. They can be used to apply medicines to a targeted area, to selectively remove substances from a targeted area, or to apply cleaning substances like Betadine. They are also used as an applicator for various cosmetics, ointments, and other substances. A related area is the use of swabs for microbiological environmental monitoring. Once taken, the swab can be streaked onto an agar plate, or the contents of the tip removed by agitation or dilution into the broth. The broth can either then be filtered or incubated and examined for microbial growth. Further, such swabs are suitable to take other clinical specimens containing viruses, including COVID-19, chlamydia, mycoplasma or ureaplasma organisms.

The term "cutting knife" as used herein is a broad term and is to be given its ordinary and customary meaning to a person of ordinary skill in the art and is not to be limited to a special or customized meaning. The term specifically may refer, without limitation, to a device with a cutting edge or blade. A cutting knife comprises a rather sharp cutting edge or blade.

The term "laser" as used herein is a broad term and is to be given its ordinary and customary meaning to a person of ordinary skill in the art and is not to be limited to a special or customized meaning. The term specifically may refer, without limitation, to a device that emits light through a process of optical amplification based on the stimulated emission of electromagnetic radiation. A laser differs from other sources of light in that it emits light which is coherent. Spatial coherence allows a laser to be focused to a tight spot, enabling applications such as laser cutting and lithography. Spatial coherence also allows a laser beam to stay narrow over great distances (collimation), enabling applications such as laser pointers and lidar. Lasers can also have high temporal coherence, which allows them to emit light with a very narrow spectrum, i.e., they can emit a single color of light. Alternatively, temporal coherence can be used to produce pulses of light with a broad spectrum but durations as short as a femtosecond ("ultrashort pulses").

The term "cutting wire" as used herein is a broad term and is to be given its ordinary and customary meaning to a person of ordinary skill in the art and is not to be limited to a special or customized meaning. The term specifically may refer, without limitation, to a rather thin wire having a thickness or diameter of 500 µm or less such as 200 µm, 100 µm or even 80 µm.

The term "aliquot" as used herein is a broad term and is to be given its ordinary and customary meaning to a person of ordinary skill in the art and is not to be limited to a special or customized meaning. The term specifically may refer, without limitation, to an exact portion of a sample or total amount of a liquid, e.g. exactly 25mL of water taken from 250 mL.

The term "aliquoting device" as used herein is a broad term and is to be given its ordinary and customary meaning to a person of ordinary skill in the art and is not to be limited to a special or customized meaning. The term specifically may refer, without limitation, to any device that is configured to take an aliquot from a sample such as a laboratory sample.

The term "inactivation fluid" as used herein is a broad term and is to be given its ordinary and customary meaning to a person of ordinary skill in the art and is not to be limited to a special or customized meaning. The term specifically may refer, without limitation, to any fluid such as a gas and/or a liquid that is configured to stop bacteria and/or viruses in a given sample from contaminating the desired product either by removing bacteria and/or viruses completely or rendering them non-infectious. For example viral inactivation fluids render viruses unable to infect. Many viruses contain lipid or protein coats that can be inactivated by chemical alteration. Viral inactivation is different from viral removal because, in the former process, the surface chemistry of the virus is altered and in many cases the (now non-infective) viral particles remain in the final product. Rather than simply rendering the virus inactive, some viral inactivation processes actually denature the virus completely. Viral inactivation is used widely in the blood plasma industry.

The term "lysis fluid" as used herein is a broad term and is to be given its ordinary and customary meaning to a person of ordinary skill in the art and is not to be limited to a special or customized meaning. The term specifically may refer, without limitation, to any fluid such as a gas and/or a liquid that is configured to break down of the membrane of a cell, often by viral, enzymic, or osmotic mechanisms that compromise its integrity. A fluid containing the contents of lysed cells is called a lysate. In molecular biology, biochemistry, and cell biology laboratories, cell cultures may be subjected to lysis in the process of purifying their components, as in protein purification, DNA extraction, RNA extraction, or in purifying organelles. Many species of bacteria are subject to lysis by the enzyme lysozyme, found in animal saliva, egg white, and other secretions. Phage lytic enzymes (lysins) produced during bacteriophage infection are responsible for the ability of these viruses to lyse bacterial cells. Penicillin and related β-lactam antibiotics cause the death of bacteria through enzyme-mediated lysis that occurs after the drug causes the bacterium to form a defective cell wall. If the cell wall is completely lost and the penicillin was used on grampositive bacteria, then the bacterium is referred to as a protoplast, but if penicillin was used on gram-negative bacteria, then it is called a spheroplast. Particularly, a lysis fluid is configured to deteriorate/solubilize the proteins and lipids present within the membrane of targeted cells.

The term "dispensing device" as used herein is a broad term and is to be given its ordinary and customary meaning to a person of ordinary skill in the art and is not to be limited to a special or customized meaning. The term specifically may refer, without limitation, to any device that is designed to release a specific amount of its content. The content in the sense of the present disclosure may particularly refer to a fluid and more particularly to a liquid. The content is more particularly an inactivation and/or lysis fluid.

The term "sealing device" as used herein is a broad term and is to be given its ordinary and customary meaning to a person of ordinary skill in the art and is not to be limited to a special or customized meaning. The term specifically may refer, without limitation, to any device that is configured to provide a laboratory sample container with a sealing. Particularly, such a sealing is impermeable for bacteria and/or viruses. The sealing is put or disposed onto the laboratory sample container in a manner so as to close its opening.

The term "recappimg device" as used herein is a broad term and is to be given its ordinary and customary meaning to a person of ordinary skill in the art and is not to be limited to a special or customized meaning. The term specifically may refer, without limitation, to any device that is configured to provide a laboratory sample container with a cap. Such a cap may be impermeable for bacteria and/or viruses. The cap is put or disposed onto the laboratory sample container in a manner so as to close its opening.

The term "sterilizing device" as used herein is a broad term and is to be given its ordinary and customary meaning to a person of ordinary skill in the art and is not to be limited to a special or customized meaning. The term specifically may refer, without limitation, to any device that is configured to remove, kill, or deactivate all forms of life, in particular referring to microorganisms such as fungi, bacteria, spores, viruses, unicellular eukaryotic organisms such as Plasmodium, etc., and other biological agents like prions present in a specific surface, object or fluid, for example biological culture media. Sterilization by such a device can be achieved through various means, including heat, chemicals, irradiation, high pressure, and filtration. Sterilization is distinct from disinfection, sanitization, and pasteurization, in that those methods reduce rather than eliminate all forms of life and biological agents present. After sterilization, an object is referred to as being sterile or aseptic.

The term "UV light source" as used herein is a broad term and is to be given its ordinary and customary meaning to a person of ordinary skill in the art and is not to be limited to a special or customized meaning. The term specifically may refer, without limitation, to a light source that is configured to emit ultraviolet (UV) light. Ultraviolet light is useful for sterilization of surfaces and some transparent objects. The UV light may particularly be emitted from a germicidal lamp. A germicidal lamp is an electric light source that produces ultraviolet C (UVC) light. This short-wave ultraviolet light disrupts DNA base pairing, causing formation of pyrimidine dimers, and leads to the inactivation of bacteria, viruses, and protozoa. It can also be used to produce ozone for water disinfection. They are used in ultraviolet germicidal irradiation (UVGI). There are four common types available: Low-pressure mercury lamps, high-pressure mercury lamps, excimer lamps and LEDs.

Further disclosed and proposed herein is a computer program, not being part of the present invention, including computer-executable instructions for performing the method in one or more of the embodiments enclosed herein when the program is executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps as indicated above may be performed by using a computer or a computer network, preferably by using a computer program.

Further disclosed and proposed herein is a computer program product, not being part of the present invention, having program code means, in order to perform the method in one or more of the embodiments enclosed herein when the program is executed on a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the method according to one or more of the embodiments disclosed herein.

Further disclosed and proposed herein is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing the method according to one or more of the embodiments disclosed herein.

Referring to the computer-implemented apsects, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:
- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

### Short description of the Figures

Further optional features and embodiments will be disclosed in more detail in the subsequent description of embodiments, preferably in conjunction with the dependent claims.

The scope of the invention is not restricted by the preferred embodiments. The embodiments are schematically depicted in the Figures. Therein, identical reference numbers in these Figures refer to identical or functionally comparable elements.

In the Figures:
- Figure 1: shows a perspective view of a preanalytic system according to a first embodiment of the present invention;
- Figure 2: shows another perspective view of the preanalytic system according to the first embodiment of the present invention;
- Figure 3: shows a dispensing device of the preanalytic system according to the first embodiment of the present invention;
- Figure 4: shows a sealing device of the preanalytic system according to the first embodiment of the present invention;
- Figure 5: shows a cutting device of a preanalytic system according to a second embodiment of the present invention.

### Detailed description of the embodiments

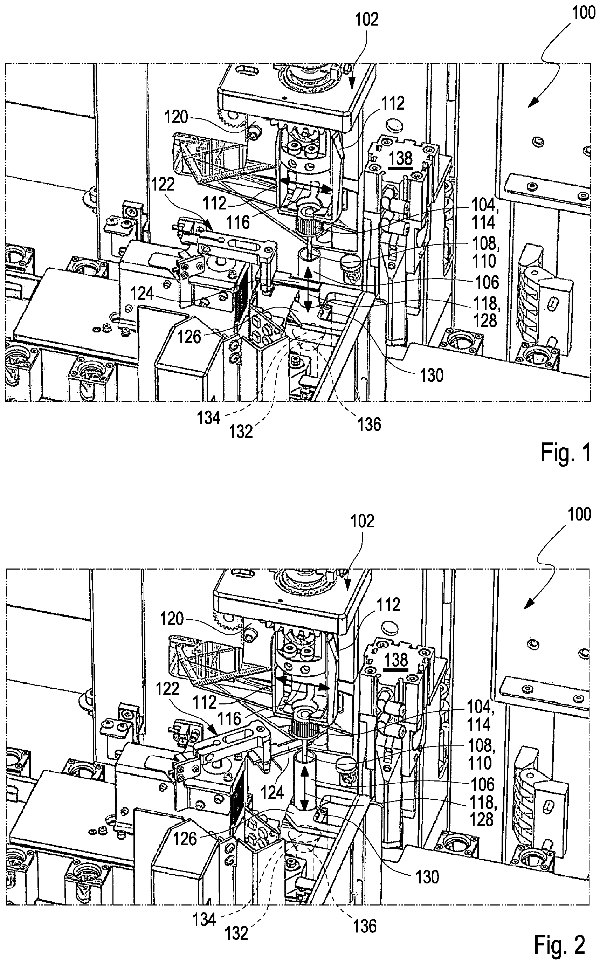

Figure 1 shows a perspective view of a preanalytic system 100 according to a first embodiment of the present invention. Figure 2 shows another perspective view of the preanalytic system 100 according to the first embodiment of the present invention. The preanalytic system 100 comprises a cap gripper 102. The cap gripper 102 is configured to grip a cap 104 of a laboratory sample container 106. The cap 104 is connected to a rod 108 of a swab 110 located within the laboratory sample container 106. The cap gripper 102 comprises at least two gripping fingers 112 configured to grip the cap 104. Particularly, the gripping fingers 112 are configured to grip the cap 104 at an outer circumferential surface 114 thereof. In a different embodiment, the cap gripper 102 may comprise at least three, four, five, six or more gripping fingers 112. Both gripping fingers 112 are displaceable between a holding position and a release position in or along a radial direction 116. In a different embodiment, it may be sufficient that only one of the at least two gripping fingers 112 may be displaceable between the holding position and the release position in the radial direction 116. In the holding position, the gripping fingers 112 hold or fix the cap 104. In the release position, the cap 104 is released by the gripping fingers 112.

The cap gripper 102 is further configured to release the cap 104 from the laboratory sample container 106. Particularly, the cap gripper 102 is further configured to release the cap 104 from the laboratory sample container 106 with the swab 110 remaining located within the laboratory sample container 106. As shown in Figure 1, the laboratory sample container extends 106 along a longitudinal direction 118. The cap gripper 102 is moveable in a direction parallel to the longitudinal direction 118. Particularly, the cap gripper 102 is moveable in the direction parallel to the longitudinal direction 118 with the cap 104 gripped so as to lift the cap 104 from the laboratory sample container 106. For this purpose, the preanalytic system 100 comprises a lifting device 120, which is configured to linearly move the cap gripper 102. For example, the lifting device 120 is configured to raise and lower the cap gripper 102 such as in an upwards and downwards movement. It has to be noted that it is sufficient for the lifting device 120 to raise the cap gripper 102 a rather short distance relative to the laboratory sample container 106 such as 2 to 5 mm in order to lift the cap 104 from the laboratory sample container 106.

The preanalytic system 100 further comprises a cutting device 122 configured to cut the rod 108 of the swab 110 when the cap 104 is in a position released from the laboratory sample container 106. Particularly, the cutting device 122 is configured to cut the rod 108 of the swab 110 such that the swab 110 together with the cut rod 108 remains located within the laboratory sample container 106. In the present embodiment, the cutting device 122 is pivotable relative to the cap gripper 102. Particularly, the cutting device 122 comprises a cutting knife 124. The cutting knife 124 may be a heated cutting knife. The cutting knife 124 is heated to 60 degree Celsius or above such as between 60 and 70 degree or between 65 and 85 degree. The cutting knife 124 is pivotable within a plane perpendicular to the longitudinal direction 118 or the direction in which the cap gripper 102 is moveable. For example, the cutting knife 122 is pivotable at an angle of substantially 90° within a plane perpendicular to the longitudinal direction 118 or the direction in which the cap gripper 102 is moveable. Particularly, the cutting device 122 is pivotable between a first or retracted position shown in Figure 1 and a second or pivoted position shown in Figure 2. Thereby, the cutting knife 124 is integrally or concertedly pivoted. In another embodiment, the cutting device 122 is rotatable, linearly moveable or stationary relative to the cap gripper 102.

The preanalytic system 100 further comprises a container holder 126 configured to hold the laboratory sample container 106. The container holder 126 is disposed at a position opposite to the cap gripper 102 if seen along a common axial direction 128, which preferably is parallel to the longitudinal direction 118. The container holder 126 holds the laboratory sample container 106, in particular in such a way that a longitudinal axis of the laboratory sample container 106 in form of the tube coincidences with the common axial direction 128. Particularly, the container holder 126 is configured to hold the laboratory sample container 106 at or adjacent to the bottom end thereof. The container holder 126 may be embodied as a gripper. Further, the container holder 126 and the cap gripper 102 with its gripping fingers 112 are displaceable relatively to each other in the common axial direction 128. Particularly, the container holder 126 and the cap gripper 102 with its gripping fingers 112 may be displaceable relatively to each other in the common axial direction 128 such that the held cap 104 is pulled away from the laboratory sample container 106. In the shown embodiment, the cap gripper 102 is arranged vertically above the container holder 126 such that, after the release of the cap 104, the open end of the laboratory sample container 106 is vertically above the rest of the laboratory sample container 106. In particular, the cap gripper 102 and the container holder 126 are oriented in such a way, that the common axial direction 128 corresponds to the vertical.

The preanalytic system 100 further comprises a waste container 130. The waste container 130 is located below the cap gripper 102 if seen in a direction of gravity which corresponds to the common axial direction 128. The waste container 130 comprises a funnel 132 having a wide end 134 and a narrow end 136. The funnel 132 is arranged such that the wide end 134 faces the cap gripper 102. With this arrangement, the cap gripper 102 is configured to dispose the cap 104 cut off from the swab 110 into the waste container 130. With this construction, after the cap 104 has been cut off from the rod 108 of the swab 110 and further transport or conveying of the laboratory sample container 106, the cap 104 may be released by the gripping fingers 112. Then, it may come off the gripping fingers 112, in particular it may fall down due to gravity.

The preanalytic system 100 further comprises an aliquoting device 138 configured to take an aliquot from a sample of the laboratory sample container 106. The aliquoting device 138 is configured to dispense the aliquot from the sample of the laboratory sample container 106 into an aliquot container such as an aliquot tube. The aliquot tube may then be further processed such as being analyzed.

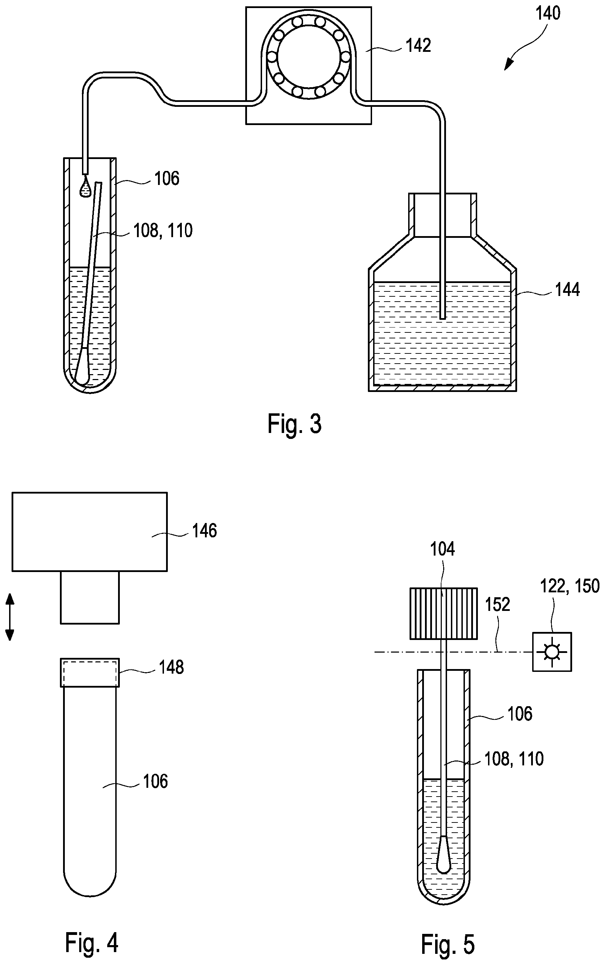

Figure 3 shows a dispensing device 140 of the preanalytic system 100 according to the first embodiment of the present invention. Thus, the preanalytic system 100 further comprises a dispensing device 140. The dispensing device 140 is configured to dispense an inactivation fluid and/or lysis fluid into the laboratory sample container 106. Particularly, the dispensing device 140 is configured to dispense a predetermined amount of the inactivation fluid and/or lysis fluid into the laboratory sample container 106. For this purpose, the dispensing device 140 comprises a pump 142 configured to pump the predetermined amount of the inactivation fluid and/or lysis fluid into the laboratory sample container 106. The pump 142 may be a roller pump. The inactivation fluid and/or lysis fluid may be provided in an inactivation fluid and/or lysis fluid reservoir 144 in fluid communication with the pump 142.

Figure 4 shows a sealing device 146 of the preanalytic system 100 according to the first embodiment of the present invention. Thus, the preanalytic system 100 may optionally further comprise a sealing device 146. The sealing device 146 is configured to provide the laboratory sample container 106 with a sealing 148. Alternatively, the preanalytical system 100 may comprise a recapping device configured to provide the laboratory sample container 106 with a cap for closing the opening thereof.

Optionally, the preanalytic system 100 may further comprise a sterilizing device (not shown in detail) configured to sterilize the laboratory sample container 106. The sterilizing device may comprise an UV light source.

Hereinafter, the operation of the preanalytic system 100, particularly of the cap gripper 102 and the cutting device 122 will be described in further detail. A laboratory sample container 106 is provided which is closed by a cap 104. The laboratory sample container 106 includes or carries a swab 110. A rod 108 of the swab 110 is connected to the cap 104. The laboratory sample container 106 is transported or conveyed within the preanalytic system 100 or to the container holder 126 by a conveying device (not shown in detail). The container holder 126 holds the laboratory sample container 106 in its position. Particularly, the container holder 126 holds the laboratory sample container 106 at or adjacent to the bottom end thereof. Then, the cap gripper 102 is lowered by means of the lifting device 120 so as to approach the container holder 126. In the lowered position of the cap gripper 102, the gripping fingers 112 are displaced from the holding position into the release position in or along the radial direction 116. Thus, the cap gripper 102 grips the cap 104. Subsequently, the cap gripper 102 is moved in the direction parallel to the longitudinal direction 118 with the cap 104 gripped so as to lift the cap 104 from the laboratory sample container 106. With other words, the cap gripper 102 is raised a predetermined and rather short distance away from the laboratory sample container 106 by means of the lifting device 120 while the cap gripper 102 still grips the cap 104. Thereby, the cap 104 is released from the laboratory sample container 106. Particularly, the cap gripper 102 releases the cap 104 from the laboratory sample container 106 with the swab 110 remaining located within the laboratory sample container 106.

When the cap 104 is in a position released from the laboratory sample container 106, the cutting device 122 cuts the rod 108 of the swab 110. Particularly, the cutting device 122 cuts the rod 108 of the swab 110 such that the swab 110 together with the cut rod 108 remains located within the laboratory sample container 106. For this purpose, the cutting device 122 and the cutting knife pivots from the first position shown in Figure 1 into the second position shown in Figure 2 so as to cut the rod 110 at a position adjacent the cap 104. Thereby, the swab 110 slides back into the laboratory sample container 106 while the cap gripper 102 still grips the cap 104.

Subsequently, the laboratory sample container 106 is released from the container holder 126 and further conveyed by means of the conveying device. Further, the cap gripper 102 disposes the cap 104 cut off from the swab 110 into the waste container 130. For this purpose, the gripping fingers 112 are displaced from the holding position into the release position and the cap 104 is released by the gripping fingers 112. Particularly, the cap 104 comes off the gripping fingers 112, in particular it may fall down due to gravity into the waste container 130.

After cutting the rod 108 of the swab 110, the laboratory sample container may be further conveyed to the aliquoting device 138. The aliquoting device 138 takes an aliquot from a sample of the laboratory sample container 106. Further, the aliquoting device 138 may dispense the aliquot from the sample of the laboratory sample container 106 into an aliquot container such as an aliquot tube. The aliquot tube may then be further processed such as being analyzed. After aliquoting, the dispensing device 140 dispenses an inactivation fluid and/or lysis fluid into the laboratory sample container 106. Particularly, the dispensing device 140 dispenses a predetermined amount of the inactivation fluid and/or lysis fluid into the laboratory sample container 106. For this purpose, the dispensing device 140 operates the pump 142 in order to pump the predetermined amount of the inactivation fluid and/or lysis fluid from the inactivation fluid and/or lysis fluid reservoir 144 into the laboratory sample container 106. The inactivation fluid and/or lysis fluid may be provided in an inactivation fluid and/or lysis fluid reservoir 144 in fluid communication with the pump 142. The pump 142 may be a roller pump. After the dispensing process, the laboratory sample container 106 may be further conveyed to the sealing device 146. There, the laboratory sample container is provided with a sealing 148. Subsequently, the sealed laboratory sample container 106 may be sorting into a waste rack (not shown in detail) on an output area of the preanalytic system 100.

Figure 5 shows a cutting device 122 of a preanalytic system 100 according to a second embodiment of the present invention. Hereinafter, only the differences from the first embodiment will be explained and like constructional members are indicated by like reference numerals. According to the second embodiment, the cutting device 122 comprises a laser 150. The laser 150 is moveable within a plane perpendicular to the longitudinal direction 118 or the direction in which the cap gripper 102 is moveable. The laser 150 may be linearly moveable within the plane perpendicular to the longitudinal direction 118 or the direction in which the cap gripper 102 is moveable. Alternatively, the laser 150 may be pivotable within the plane perpendicular to the longitudinal direction 118 or the direction in which the cap gripper 102 is moveable. For example, the laser 150 may be pivotable at an angle of substantially 90° within a plane perpendicular to the longitudinal direction 118 or the direction in which the cap gripper 102 is moveable. It is explicitly stated that the cutting device 122 according to the first and second embodiments may alternatively or in addition comprise a cutting wire. The operation principle of the preanalytic system 100 according to the second embodiment is substantially identical to the operation principle of the preanalytic system 100 according to the first embodiment except for the rod 108 of the swab 110 being cut by means of the laser 150 emitting laser light 152 towards the rod 108. Regarding the remaining operation of the preanalytic system 100 according to the second embodiment, reference is made to the above explanations of the operation of the preanalytic system 100 according to the first embodiment.

### List of reference numbers

- 100: preanalytic system
- 102: cap gripper
- 104: cap
- 106: laboratory sample container
- 108: rod
- 110: swab
- 112: gripping finger
- 114: outer circumferential surface
- 116: radial direction
- 118: longitudinal direction
- 120: lifting device
- 122: cutting device
- 124: cutting knife
- 126: container holder
- 128: common axial direction
- 130: waste container
- 132: funnel
- 134: wide end
- 136: narrow end
- 138: aliquoting device
- 140: dispensing device
- 142: pump
- 144: inactivation fluid and/or lysis fluid reservoir
- 146: sealing device
- 148: sealing
- 150: laser
- 152: laser light

## Claims

1. A preanalytic system (100), comprising
a cap gripper (102) configured to grip a cap (104) of a laboratory sample container (106), wherein the cap (104) is connected to a rod (108) of a swab (110) located within the laboratory sample container (106), wherein the cap gripper (102) is further configured to release the cap (104) from the laboratory sample container (106),
**characterized in that**
the preanalytic system (100) further comprises a cutting device (122) configured to cut the rod (108) of the swab (110) when the cap (104) is in a position released from the laboratory sample container (106).

2. The preanalytic system (100) according to the preceding claim, wherein the cap gripper (102) is further configured to release the cap (104) from the laboratory sample container (106) with the swab (110) remaining located within the laboratory sample container (106).

3. The preanalytic system (100) according to any preceding claim, wherein the cutting device (122) is configured to cut the rod (108) of the swab (110) such that the swab (110) together with the cut rod (108) remains located within the laboratory sample container (106).

4. The preanalytic system (100) according to any preceding claim, wherein the laboratory sample container (106) extends along a longitudinal direction (118), wherein the cap gripper (102) is moveable in a direction parallel to the longitudinal direction (118).

5. The preanalytic system (100) according to the preceding claim, wherein the cap gripper (102) is moveable in the direction parallel to the longitudinal direction (118) with the cap (104) gripped so as to lift the cap (104) from the laboratory sample container (106).

6. The preanalytic system (100) according to any preceding claim, wherein the cap gripper (102) comprises at least two gripping fingers (112) configured to grip the cap (104).

7. The preanalytic system (100) according to the preceding claim, wherein the gripping fingers (112) are configured to grip the cap (104) at an outer circumferential surface (114) thereof.

8. The preanalytic system (100) according to any preceding claim, wherein the cutting device (122) is rotatable, pivotable, linearly moveable or stationary relative to the cap gripper (102).

9. The preanalytic system (100) according to any preceding claim, wherein the cutting device (122) comprises a laser (150), a cutting wire and/or a cutting knife (124), particularly a heated cutting knife, in particular heated to 60 degree Celsius or above, preferably between 60 and 70 degree or between 65 and 85 degree.

10. The preanalytic system (100) according to any preceding claim, further comprising a container holder (128) configured to hold the laboratory sample container (106).

11. The preanalytic system (100) according to any preceding claim, further comprising a dispensing device (140) configured to dispense an inactivation fluid and/or lysis fluid into the laboratory sample container (106).

12. The preanalytic system (100) according to the preceding claim, wherein the dispensing device (140) is configured to dispense a predetermined amount of the inactivation fluid and/or lysis fluid into the laboratory sample container (106).

13. The preanalytic system (100) according to the preceding claim, wherein the dispensing device (140) comprises a pump (142), particularly a roller pump, configured to pump the predetermined amount of the inactivation fluid and/or lysis fluid into the laboratory sample container (106).

14. The preanalytic system (100) according to any preceding claim, further comprising a sealing device (146) or recapping device configured to provide the laboratory sample container (106) with a sealing (148) or a cap.

15. The preanalytic system (100) according to any preceding claim, further comprising a sterilizing device configured to sterilize the laboratory sample container (106) in particular the sterilizing device comprises an UV light source.

## Patentansprüche

1. Präanalytisches System (100), umfassend
einen Kappengreifer (102), der zum Greifen einer Kappe (104) eines Laborprobenbehälters (106) ausgebildet ist, wobei die Kappe (104) mit einem Stab (108) eines Tupfers (110) verbunden ist, der sich in dem Laborprobenbehälter (106) befindet, wobei der Kappengreifer (102) ferner zum Abnehmen der Kappe (104) von dem Laborprobenbehälter (106) ausgebildet ist,
**dadurch gekennzeichnet, dass**
das präanalytische System (100) ferner eine Schneidvorrichtung (122) umfasst, die dazu ausgebildet ist, den Stab (108) des Tupfers (110) zu schneiden, wenn sich die Kappe (104) in einer von dem Laborprobenbehälter (106) abgenommenen Position befindet.

2. Präanalytisches System (100) nach dem vorstehenden Anspruch, wobei der Kappengreifer (102) ferner zum Abnehmen der Kappe (104) von dem Laborprobenbehälter (106) ausgebildet ist, wobei der Tupfer (110) in dem Laborprobenbehälter (106) platziert bleibt.

3. Präanalytisches System (100) nach einem vorstehenden Anspruch, wobei die Schneidvorrichtung (122) derart zum Schneiden des Stabes (108) des Tupfers (110) ausgebildet ist, dass der Tupfer (110) zusammen mit dem geschnittenen Stab (108) in dem Laborprobenbehälter (106) platziert bleibt.

4. Präanalytisches System (100) nach einem vorstehenden Anspruch, wobei der Laborprobenbehälter (106) sich entlang einer Längsrichtung (118) erstreckt, wobei der Kappengreifer (102) in einer Richtung parallel zu der Längsrichtung (118) beweglich ist.

5. Präanalytisches System (100) nach dem vorstehenden Anspruch, wobei der Kappengreifer (102) in der Richtung parallel zur Längsrichtung (118) beweglich ist, wobei die Kappe (104) so gegriffen wird, dass die Kappe (104) von dem Laborprobenbehälter (106) abgehoben wird.

6. Präanalytisches System (100) nach einem vorstehenden Anspruch, wobei der Kappengreifer (102) mindestens zwei Greiffinger (112) umfasst, die zum Greifen der Kappe (104) ausgebildet sind.

7. Präanalytisches System (100) nach dem vorstehenden Anspruch, wobei die Greiffinger (112) zum Greifen der Kappe (104) an einer Außenumfangsfläche (114) davon ausgebildet sind.

8. Präanalytisches System (100) nach einem vorstehenden Anspruch, wobei die Schneidvorrichtung (122) relativ zu dem Kappengreifer (102) drehbar, schwenkbar, linear beweglich oder feststehend ist.

9. Präanalytisches System (100) nach einem vorstehenden Anspruch, wobei die Schneidvorrichtung (122) einen Laser (150), einen Schneiddraht und/oder ein Schneidmesser (124) umfasst, insbesondere ein erhitztes Schneidmesser, insbesondere erhitzt auf 60 Grad Celsius oder darüber, vorzugsweise zwischen 60 und 70 Grad oder zwischen 65 und 85 Grad.

10. Präanalytisches System (100) nach einem vorstehenden Anspruch, ferner umfassend eine Behälterhalterung (128), die zum Halten des Laborprobenbehälters (106) ausgebildet ist.

11. Präanalytisches System (100) nach einem vorstehenden Anspruch, ferner umfassend eine Dosiervorrichtung (140), die zum Dosieren einer Inaktivierungsflüssigkeit und/oder Lyseflüssigkeit in den Laborprobenbehälter (106) ausgebildet ist.

12. Präanalytisches System (100) nach dem vorstehenden Anspruch, wobei die Dosiervorrichtung (140) zum Dosieren einer vorbestimmten Menge der Inaktivierungsflüssigkeit und/oder Lyseflüssigkeit in den Laborprobenbehälter (106) ausgebildet ist.

13. Präanalytisches System (100) nach dem vorstehenden Anspruch, wobei die Dosiervorrichtung (140) eine Pumpe (142), insbesondere eine Rollenpumpe, umfasst, die zum Pumpen der vorbestimmten Menge der Inaktivierungsflüssigkeit und/oder Lyseflüssigkeit in den Laborprobenbehälter (106) ausgebildet ist.

14. Präanalytisches System (100) nach einem vorstehenden Anspruch, ferner umfassend eine Abdichtungsvorrichtung (146) oder Wiederverkappungsvorrichtung, die zum Versehen des Laborprobenbehälters (106) mit einer Abdichtung (148) oder einer Kappe ausgebildet ist.

15. Präanalytisches System (100) nach einem vorstehenden Anspruch, ferner umfassend eine Sterilisationsvorrichtung, die zum Sterilisieren des Laborprobenbehälters (106) ausgebildet ist, insbesondere wobei die Sterilisationsvorrichtung eine UV-Lichtquelle umfasst.

## Revendications

1. Système de préanalyse (100), comprenant
un préhenseur de capuchon (102) conçu pour saisir un capuchon (104) d'un récipient d'échantillons de laboratoire (106), dans lequel le capuchon (104) est relié à une tige (108) d'un écouvillon (110) situé à l'intérieur du récipient d'échantillons de laboratoire (106), dans lequel le préhenseur de capuchon (102) est en outre conçu pour libérer le capuchon (104) du récipient d'échantillons de laboratoire (106),
**caractérisé en ce que**
le système de préanalyse (100) comprend en outre un dispositif de coupe (122) conçu pour couper la tige (108) de l'écouvillon (110) lorsque le capuchon (104) est dans une position libérée du récipient d'échantillons de laboratoire (106).

2. Système de préanalyse (100) selon la revendication précédente, dans lequel le préhenseur de capuchon (102) est en outre conçu pour libérer le capuchon (104) du récipient d'échantillons de laboratoire (106) avec l'écouvillon (110) restant situé à l'intérieur du récipient d'échantillons de laboratoire (106).

3. Système de préanalyse (100) selon l'une quelconque des revendications précédentes, dans lequel le dispositif de coupe (122) est conçu pour couper la tige (108) de l'écouvillon (110) de telle sorte que l'écouvillon (110) conjointement avec la tige coupée (108) reste situé à l'intérieur du récipient d'échantillons de laboratoire (106).

4. Système de préanalyse (100) selon l'une quelconque des revendications précédentes, dans lequel le récipient d'échantillons de laboratoire (106) s'étend le long d'une direction longitudinale (118), dans lequel le préhenseur de capuchon (102) peut être déplacé dans une direction parallèle à la direction longitudinale (118).

5. Système de préanalyse (100) selon la revendication précédente, dans lequel le préhenseur de capuchon (102) peut être déplacé dans la direction parallèle à la direction longitudinale (118) avec le capuchon (104) saisi de manière à soulever le capuchon (104) du récipient d'échantillons de laboratoire (106).

6. Système de préanalyse (100) selon l'une quelconque des revendications précédentes, dans lequel le préhenseur de capuchon (102) comprend au moins deux doigts de préhension (112) conçus pour saisir le capuchon (104).

7. Système de préanalyse (100) selon la revendication précédente, dans lequel les doigts de préhension (112) sont conçus pour saisir le capuchon (104) au niveau d'une surface circonférentielle extérieure (114) de celui-ci.

8. Système de préanalyse (100) selon l'une quelconque des revendications précédentes, dans lequel le dispositif de coupe (122) peut être tourné, pivoté, déplacé de manière linéaire ou stationnaire par rapport au préhenseur de capuchon (102).

9. Système de préanalyse (100) selon l'une quelconque des revendications précédentes, dans lequel le dispositif de coupe (122) comprend un laser (150), un fil de coupe et/ou un couteau de coupe (124), en particulier un couteau de coupe chauffé, en particulier chauffé à 60 degrés Celsius ou plus, de préférence entre 60 et 70 degrés ou entre 65 et 85 degrés.

10. Système de préanalyse (100) selon l'une quelconque des revendications précédentes, comprenant en outre un porte-récipient (128) conçu pour porter le récipient d'échantillons de laboratoire (106).

11. Système de préanalyse (100) selon l'une quelconque des revendications précédentes, comprenant en outre un dispositif de distribution (140) conçu pour distribuer un fluide d'inactivation et/ou un fluide de lyse dans le récipient d'échantillons de laboratoire (106).

12. Système de préanalyse (100) selon la revendication précédente, dans lequel le dispositif de distribution (140) est conçu pour distribuer une quantité prédéterminée du fluide d'inactivation et/ou du fluide de lyse dans le récipient d'échantillons de laboratoire (106).

13. Système de préanalyse (100) selon la revendication précédente, dans lequel le dispositif de distribution (140) comprend une pompe (142), en particulier une pompe à rouleau, conçue pour pomper la quantité prédéterminée du fluide d'inactivation et/ou du fluide de lyse dans le récipient d'échantillons de laboratoire (106).

14. Système de préanalyse (100) selon l'une quelconque des revendications précédentes, comprenant en outre un dispositif d'étanchéification (146) ou un dispositif de recapuchonnage conçu pour fournir au récipient d'échantillons de laboratoire (106) un joint d'étanchéité (148) ou un capuchon.

15. Système de préanalyse (100) selon l'une quelconque des revendications précédentes, comprenant en outre un dispositif de stérilisation conçu pour stériliser le récipient d'échantillons de laboratoire (106) en particulier le dispositif de stérilisation comprend une source de lumière UV.
